# EUROPEAN PATENT SPECIFICATION

(11) **EP 3 952 810 B1**
(45) Date of publication and mention of the grant of the patent: **31.07.2024**
(21) Application number: 20718652.9
(22) Date of filing: 09.04.2020
(51) Int. Cl.: A61F 13/42

(54) **AUTOMATED INCONTINENCE MONITORING DIAPER, SYSTEM AND METHOD**
AUTOMATISCHE INKONTINENZÜBERWACHUNGSWINDEL, SYSTEM UND VERFAHREN
COUCHE, SYSTÈME ET PROCÉDÉ DE SURVEILLANCE AUTOMATISÉE DE L'INCONTINENCE

(30) Priority: 10.04.2019 DE 102019002636
(43) Date of publication of application: 16.02.2022
(73) Proprietor: Advosense GmbH, 10115 Berlin (DE)
(72) Inventor: WEBB, Erin, 10709 Berlin (DE); VIDUKA, Martina, 12059 Berlin (DE); CASTELL, Jasper, 10405 Berlin (DE)
(74) Representative: Stellbrink & Partner Patentanwälte mbB
(86) International application number: PCT/EP2020/060264
(87) International publication number: WO 2020/208193

(56) References cited:
- DE-A1- 102010 018 107
- DE-U1- 202013 101 920
- US-A1- 2008 074 274

## Description

### Field

The present invention relates to a diaper, a system and a method for an efficient, reliable and automated incontinence monitoring.

### Introduction

More than 200 million people live with urinary incontinence (UI) globally. In addition to affecting quality of life, UI is a leading risk factor for preventable pressure ulcers, falls, and urinary tract infections because skin exposure to urine due to infrequent adult brief changes can predispose the skin to breakdown, which can cause significant pain, suffering, and prolonged hospital stays.

By reducing costly hospital acquired conditions, reducing waste in inventory management, increasing quality of life for patients and maximizing efficient workflow for clinicians - such as nurses - we are changing the management of incontinence care. The solution unlocks the core values as stated by the clinician as it is discrete for patients, provides higher quality patient care, lowers costs, and enables seamless workflow integration.

The worldwide market for urinary incontinence products is expected to grow at a CAGR of roughly 9.5% over the next five years, reaching 16 Billion US dollars in 2024. Applicant's market research has resulted in a serviceable market size expectation of approximately 1.2 million patients in the industrial societies of the United States, Canada and Germany as reference markets. As these patients suffer from chronic incontinence, an average of 6-10 brief changes every day is required for the remaining lifetime of the patients.

Each country's market is unique in terms of how the health system is structured, with implications for how the applicant would go-to-market. Each country has different ways to insure individuals, reimburse hospitals and nursing homes, pay for briefs, and protect data privacy. Nevertheless, all three markets are experiencing an aging population, staff shortages in nurses and all have university hospitals and nursing homes, which are more likely and have the capacity and willingness to incorporate cutting-edge technology.

EP 1398014 A2 is directed manufacturing method of an electronic diaper is to provide a sensor feeding band or box supplying a diaper body with a sensor which will be in advance attached on the upper or the lower dry layer of the diaper body for carrying out a sequent process of automatically manufacturing an electronic diaper. The electronic diaper includes an upper and lower dry layer and an absorbing layer sandwiched between the two dry layers, a sensor attached on the upper or the lower dry layer. The sensor has a connecting member for connecting with a controller. Sensing the diaper body wetted, the sensor will give a signal to the controller which will immediately give out warning signals to remind a nurse that the diaper is wet and needs to be replaced with a new one so that a user may feel comfortable in using the electronic diaper.

CN 101472543-B relates to a moisture monitoring system for monitoring wetness in one or more absorbent articles, and includes an input for receiving one or more sensor signals indicative of a presence of wetness in an absorbent article, a processor for processing the one or more sensor signals and for performing an analysis of the signals to characterize wetness events occurring in an absorbent article and user interface for communicating with a user of the system. A mathematical model is used to characterize wetness events, receiving as inputs variables derived from sensor signals and optionally, patient and demographic data. The mathematical model can be configured and/or re-configured by an algorithm utilizing observation data obtained while monitoring a patient for wetness.

CN-108489533-A discloses a kind of disposable paper diaper built-in inspection and detection methods that belong to the technical field of intelligent paper diaper, including the circuit board being attached to inside paper diaper and the alarm module being placed in outside paper diaper, The circuit board is equipped with a core circuit region, a temperature sensor, a urine wet sensor group and bioelectrogenesis module. The core circuit region includes a power management module, a data centralized procurement module and a wireless transport module. The data centralized procurement module is respectively provided with a power management module, a urine wet sensor group, a wireless transport module and temperature sensor electrical connection, a power management module is electrically connected with a bioelectrogenesis module. A wireless transport module is connected with an alarm module with wireless transmission in order to reach the paper diaper and can know a little the number of wet regions and urine volume in order to judge urination by a urine self-powered generation for urination sensing.

AU-2010314813-B2 discloses a system for monitoring incontinence in one or more subjects comprises display means; input means operable by a user; one or more sensors, each sensor being associated with one or more subjects being monitored; the one or more sensors being configured to transmit signals containing continence-related data for the one or more subjects, wherein the incontinence-related data has been obtained over time from a continence sensor associated with an absorbent article worn by each respective subject; a receiver unit configured to receive signals from the one or more sensors; and processing means in communication with at least the receiver unit, the processing means including a display processor configured to process the received signals and communicate display information to the display means for display of a visual representation of continence-related information derived from continence sensors in the absorbent articles worn by the one or more subjects being monitored. The system may include a volume estimator and means for communicating to a nurse a 'risk of wetness leakage' based on e.g. an estimated volume of wetness and e.g. a pad type. Pad type may be communicated to the system automatically by way of a pad type indicator associated with the pad and/or pad/sensor combination.

US 8421636 B2 relates to a patient monitoring system according to one embodiment and includes a real-time wetness sensor configured to detect the rate of change in wetness occurring within an associated diaper, and automatically adjusting the sensitivity of the sensor to account for a wetness event unrelated to urination. A monitoring unit may be utilized in such a manner that the monitoring system controller-monitors the wetness sensor and generates data associated with detected-wetness events relative to the diaper. A wireless sensor configured with the monitoring unit and in communication with the monitoring system controller may be further utilized to send the generated data, through a host computer, to a caregiver unit having a caregiver system controller in communication with the host computer. The caregiver can then check the status of the patient, provide a service, and annotate the patient's history by transmitting the recorded observation and service provided back to the base station.

DE 20 2013 101920 U1 discloses a hygiene article for receiving, in particular, liquid bodily excretions, having an absorbent material for receiving liquid and at least one moisture sensor arranged in the absorbent material, characterized in that the moisture sensor is an RFID component.

DE 10 2010 018107 A1 discloses a diaper with feedback: diapers are changed regularly at set care intervals along with other care scopes. It can happen that a diaper that has just been changed becomes soiled shortly afterwards. After this, the patient must remain soiled until the next specified change interval. This results in bedsores and other bacterial diseases. Diapers are also changed that are not yet contaminated, resulting in unnecessary additional costs.

The before described processes are often unreliable and rather expensive with an unsatisfying outcome.

### Summary

In light of the above, it is an object of the present invention to overcome or at least alleviate the shortcomings of the prior art. More particularly, it is an object of the present invention to provide a diaper, a system and a method for an automated monitoring of incontinence.

This object is attained with the subject matter in accordance with the below description, embodiments and claims.

The present invention relates to a diaper comprising a first transmitter at a first location that is configured to get contaminated with excrement, humidity, and/or wetness, and that is configured to become inactive upon contamination with excrement, humidity, and/or wetness. The diaper further comprises a second transmitter at a second location that is configured to stay uncontaminated with excrement, humidity and/or wetness, the diaper further comprising a processing component controlling at least one of the first transmitter and the second transmitter, wherein the processing component is configured to detect a difference between signals from the first transmitter and the second transmitter.

Herein the term diaper is predominantly used. This also embraces what is also called as a brief for adults and/or elderly people.

The term transmitter is intended to mean any device or arrangement of hardware and/or software that is configured to actively and/or passively (upon excitation) transmit a signal.

The first transmitter can comprise a first humidity-sensor and/or soiling sensor. This is intended to comprise any sensor that is capable of sensing contamination of the diaper with excrements.

The term excrement is intended to comprise feces, stool, bodily fluids, such as urine, purulent drainage and/or blood.

The first transmitter can be configured to submit a signal in an uncontaminated status. Uncontaminated is intended to mean a state that the diaper does not need to be changed. It may be contaminated slightly but still be able to absorb more excrement and still avoids the user feeling uncomfortable and/or avoids adverse events for the user.

The first transmitter can be configured to be passive and to submit a signal upon remote excitation in an uncontaminated status. The remote excitation can be realized by a component that is configured for excitation of the transmitter. This component can be combined and/or integrated with another component, such as a reader of the signal of the transmitter.

The first transmitter can be configured to be short-circuited upon contamination with excrement. This can easily be done by two electrodes side-by-side and not connected with each other. As the excrement usually is conductive - at least in part - the closed by electrodes can be short-circuited by excrement. There are also other ways, such as avoiding the antenna to properly transmit the transmitter's signal, and/or aggregated and/or combined phenomena.

The first transmitter can be configured to submit a signal in an uncontaminated status in a fixed interval and/or different intervals. The interval can be pre-set. The interval can be self-adjusting according to the amount of contamination of the transmitter.

The intervals can be set from 1 to 10 min constantly and/or can be subject to change according to the time lapsed.

The first transmitter can be configured to be short-circuited upon contamination with excrement. This has the preferred advantage that a simple and inexpensive sensor can be used with a reliable information generated for use. Even in case the sensor fails this would lead to a default situation that won't harm the wearer of the diaper or brief.

The first transmitter can comprise a first humidity-sensor that is configured to be short-circuited upon contamination with excrement. As mentioned, excrement can comprise a number of fluids, moisture, stool etc.

The second transmitter can be of the same simple and inexpensive design as the first transmitter and provides an additional control of the correct working of the sensor.

Inactive means that no signal or no signal that can be compared with the normal status in a diaper without any excrement can be transmitted and/or received.

The second transmitter can comprise a second humidity-sensor. It can be sensitive with respect to humidity contained in urine or stool etc. However, as stated before and below, the sensor alternatively and/or additionally can be sensitive to other factors as well.

The second transmitter can be configured to submit a signal in an uncontaminated status.

The second transmitter can be of passive nature and can be configured to submit a signal upon remote excitation in an uncontaminated status. It can be configured to submit a signal in an uncontaminated status in intervals.

The first and the second transmitters can be further configured to submit each signal in an uncontaminated status in synchronized intervals. This can be at the same time so that a signal can be aggregated. In this case the signal strength is increased while in case just the second transmitter works, the signal is non-aggregated and lower. The signals of both transmitters can also be transmitted at different times. In this case if one of the transmitters fails, the interval between signals gets larger. This can be easily detected and an alarm representing a diaper with excrements can be initiated.

For economic reasons and/or for easier detection of the signals the interval can be pre-set. Additionally or alternatively, the intervals can also defer during one cycle of one diaper and can get shorter over time as a contamination will get more and more likely over time. The interval(s) can be self-adjusting according to the amount of contamination of the transmitter and/or according to the time lapsed. The interval between signals or signals to be expected can be from 1 to 10 min. They can be also shorter and/or longer and also between full minutes.

The second transmitter can be configured to be short-circuited upon contamination with excrement. This can easily be done by two electrodes side-by-side and not connected with each other. As the excrement usually is conductive - at least in part - the closed by electrodes can be short-circuited by excrement. There are also other ways, such as avoiding the antenna to properly transmit the transmitter's signal, and/or aggregated and/or combined phenomena.

The second transmitter can comprise of a humidity-sensor that is configured to be short-circuited upon contamination with excrement. The excrement can comprise feces, stool and/or bodily fluids, such as urine, purulent drainage and/or blood etc.

The first sensor and/or the first location can be configured to get contaminated and/or wet during use of the diaper.

The first humidity sensor can be sensitive to the contamination with humidity and/or liquid. The first sensor can be configured to determine the presence or the absence of humidity and/or liquid. The first sensor can be configured to determine the presence and/or the absence of humidity and/or liquids. The first sensor can be configured to measure humidity and/or liquids.

The second transmitter can comprise a second humidity sensor and/or any sensor that is sensitive with respect to diaper or brief contamination. The second transmitter and/or the second location can be configured to stay dry during use of the diaper.

The second sensor can be of the same kind as the first sensor. The second sensor can also be of a different kind than the first sensor. The second sensor can also be less sensitive than the first sensor.

The second sensor can be also liquid sensitive. Needless to say, that the sensor can be also sensitive to any other or additional contamination. The first sensor can be configured to determine the presence or the absence of humidity and/or liquids.

The second sensor can be configured to determine the presence or the absence of humidity and/or liquids. The second sensor can be configured to measure humidity and/or liquids. The first transmitter can be arranged in and/or at the central part and/or any absorbent part of the diaper.

The first transmitter can be arranged in and/or in the vicinity of the inside of the diaper.

The second transmitter can be arranged in and/or at peripheral part of the diaper. A distance between the first transmitter and the second transmitter can be at least 10 cm. a distance between the first transmitter and the second transmitter can be between 10 cm and 25 cm. A distance between the first transmitter and the second transmitter can be between 15 cm and 25 cm. A distance between the first transmitter and the second transmitter can be between 18 cm and 25 cm. A distance between the first transmitter and the second transmitter can also be between 20 cm and 25 cm.

The second transmitter can be arranged in and/or a closure of the diaper. The second transmitter can be arranged in and/or a fastener of the diaper. The second transmitter can be arranged at the outside and/or the vicinity of the outside of the diaper.

The first transmitter can comprise an antenna and/or can be integrated with each other. The first transmitter can comprise a first RFID component. The first transmitter can be integrated with a first RFID component.

The second transmitter can be setup as the first transmitter, e.g., can comprise a second antenna. The second transmitter can comprise a second RFID component. The second transmitter can be integrated with a second RFID component.

The first RFID component can be active and/or passive. The second transmitter can be active and/or passive. The first transmitter can further comprise a first temperature sensor. The first transmitter can be configured to transmit data corresponding to a read-out of the humidity sensor and/or the first temperature sensor. The second transmitter can further comprise a second temperature sensor. The second transmitter can be configured to transmit data corresponding to a read-out of the humidity sensor and/or the second temperature sensor.

The processing component can comprise a processing component RFID. The processing component RFID can be active. The processing component RFID can be passive as well. The latter bears the advantage that the chip can be made more simple, reliable and inexpensive.

The passive first transponder, the passive second transponder and/or the passive processing component RFID can each or in combination be configured to be excited by an external electromagnetic source.

The active and/or passive first transponder, the second transponder and/or the processing component RFID can be configured to be excited by an external HF (high frequency) and/or UHF (ultra-high frequency) field.

The processing component is configured to detect a difference between signals from the first transmitter and the second transmitter, as described.

The processing component can be configured to wirelessly transmit data corresponding to a read-out of the first transmitter and/or the second transmitter and/or a difference between the read-outs from the first transmitter and the second transmitter.

The processing component can be configured to provide an alarm signal corresponding to a read-out of the first transmitter and/or the second transmitter and/or a difference between the read-outs from the first transmitter and the second transmitter.

The processing component can be configured to provide an alarm signal corresponding to a read-out of the first transmitter and/or the second transmitter and/or a difference between the read-outs from the first transmitter and the second transmitter.

The processing component can be configured to provide an alarm signal upon reaching a pre-set threshold corresponding to a difference between the read-outs from the first transmitter and the second transmitter.

The processing component can be configured to provide an alarm signal in case an unsuccessful read-out of the first transmitter.

The present invention also relates to a system for monitoring a diaper, particularly with a diaper as described before and below. The system can comprise a receiver configured to receive any signals from the diaper. The receiver can be further configured to communicate with the diaper. This can be particularly the case if the receiver comprises and/or is combined with a component for excitation of one or more passive transmitters in the diaper.

The receiver can be configured to communicate with the diaper when the diaper is in the vicinity of the receiver. The system can further comprise an identification component that is configured to allow the receiver to only receive signals from, communicate with and/or excite transmitters of diapers applied to a relevant user. The user can be a patient, a person, an elderly person etc., as mentioned before, that is affiliated with the respective receiver. This is intended to ensure that only diapers applied are being read out and that a signal can be brought in relation to a specific person. Also IDs can be provided with the diaper transmitter(s) that are read out initially before, during or after application so that the respective user of the diaper can be identified by the system.

The receiver can be configured to communicate with the diaper when the diaper is located in the range of up to 250 cm away from the receiver, up to 200 cm away from the receiver, up to 150 cm away from the receiver, up to 100 cm away from the receiver, up to 100 cm away from the receiver, up to 75 cm away from the receiver, up to 50 cm away from the receiver, up to 25 cm away from the receiver or up to 10 cm away from the receiver. The latter distances are particularly adapted when the receiver are affiliated, attached and/or integrated into chairs, wheel chairs, beds etc.

The receiver can comprise an RFID receiver. In particular, the receiver can comprise an RFID excitation component that is configured to excite the transmitter(s) of the diaper comprising RFID(s). The RFID excitation component can be configured to excite or activate the transmitter any pre-set time interval. The time interval can be between 1 min to 10 mins, between 1 min - 8 mins, between 1 min - 6 mins or between 1 min - 5 mins.

The RFID excitation component can be configured to excite the transmitter(s) of the diaper comprising RFID(s) by electromagnetic radiation.

The system can further comprise a data processing device that is configured to control the receiver and/or to be controlled by the receiver. The data processing device can be configured to track alarms triggered. That can be stored in a database. The data processing device can be further configured to control an alarm system.

The data processing device can be configured to activate an alarm system in case of a detection of excrement, humidity and/or wetness by the diaper.

The receiver and/or processing device can be able to generate an alarm signal in case a wet or contaminated diaper is detected.

The receiver can be arranged in and/or attached at a bed, a wheelchair, a chair, a table, in a holder, at or in a wall and/or a ceiling. Other parts of the interior or furniture can also be arranged with the receiver either originally or retro-fit.

The system can further comprise an alarm component. The alarm component can be arranged originally or retro-fit in a handheld device, such as a smartphone, a tablet, in a pager, can comprise a call bell, an intercom device, can be arranged in or at a desk top computer etc.

The system can also comprise an alarm deactivation device that is configured to deactivate the alarm upon change of the contaminated diaper. The alarm deactivation device can be configured to automatically deactivate the alarm upon change of the contaminated diaper.

The alarm deactivation device can be configured to enable manual deactivation of the alarm upon change of the contaminated diaper.

The system can comprise a monitoring system configured for monitoring the number of diapers for a person per time unit. It can also comprise a monitoring system setting a monitoring alarm when the need for the change of diapers exceeds a threshold. The monitoring system can be also configured for setting a monitoring alarm when the need for the change of diapers exceeds a number of diapers for a person over time.

The present invention also relates to a method of monitoring a diaper or brief, the method making use of all the features described before and below with respect to the diaper and system in terms of respective method steps.

The invention bears the following preferred advantages and specifications:
The present invention can be particularly directed to a diaper with an active or passive sensor and method of using a respective diaper and diaper fastening. The invention concerns a diaper with an active or passive sensor, a method of using a diaper and a diaper fastener.

There is the problem that diapers which are equipped with an active or passive sensor element to inform third parties whether the diaper is wet or dry, often function poorly, as the moisture affects the sensor. If the sensor element is placed in a place that does not get wet, it can be installed within the diaper lines that are laid and this makes the production of the unit of the sensor and the sensor rather complicated and expensive.

Any transmitter, particularly the first one, can be arranged even outside the brief in case it is configured that the contamination is sensed by the transmitter. In one embodiment the first transmitter is then made inactive. In the present context the outer arrangement of the first sensor would be in accordance with the present invention.

The present invention relates to a diaper with an active or passive sensor element wherein the diaper has a first sensor at a first location of the diaper, which can get contaminated by excrement (e.g., wet by urine), and a second sensor element attached to a second place, which does not get wet when using the diaper.

As a result, two sensor elements can be used which are not connected to each other and that the failure of the first sensor does not prevent the detection of wetness, because the difference between the values transmitted by the transmitted signals can be traced to wetness in the diaper.

In addition to the sensor at the first and second location, a temperature measuring device can be provided. This enables to use the sensors or to a detected wetness determined by the sensors in the diaper of a temperature at the first sensor and preferably a temperature difference between the locations of the first and second sensor.

To distinguish between a general heat or moisture build-up and one caused by urine heat or humidity, sensors are attached to different places of the diaper. An evaluation device can in the event of failure of a sensor can be traced back to the effect of moisture.

For example, if the first sensor is located on the inside of the diaper and the second sensor element is located on the outside of the diaper, the failure of the first sensor indicates that the diaper has become wet with urine, especially if the second transmission element is functioning properly.

Technically, it is particularly easy if the second sensor is attached to a fixing adhesive strip, that is, a so-called diaper fastener. Such diaper fasteners can be usually made of a multilayer plastic material in which an active and especially a passive sensor can be integrated. It can be therefore preferably advantageous if the sensor is an RFID element.

Thus, the signals of a first sensor which can be attached to a place on the diaper that can get wet with urine and the signals of a second sensor which can be installed at a location which does not get wet when the diaper is used can be taken into account. From the difference of these signals it can be concluded that a diaper has become wet.

Therefore, it is suggested that an alarm can be triggered if no signal of the second sensor is detected.

It is advantageous if a signal is triggered when the difference between the signal strength of the first sensor and the signal strength of the second sensor is detected and when a threshold of the difference is reached.

This difference is particularly large if no signal from the first sensor and a signal from the second sensor are detected.

It is further proposed that a first temperature measuring device measures a temperature at a first location of the diaper, which gets wet with urine and a signal is triggered when a threshold value is reached. If the temperature measuring device thus prevents the exceeding of a certain temperature in the brief or diaper, it can be measured from a certain threshold and can be concluded that the diaper is soaked through and a signal is triggered.

It is particularly advantageous when a second temperature measuring device is used to measure a second temperature at a second location of the diaper, which usually doesn't get wet by urine rather not wet. If a threshold is exceeded, the difference between the first and second temperature triggers a signal.

E.g., when the wearer of a diaper wearer gets fever, both temperature measuring devices measure the same value or similar values. However, if a temperature measuring device is wetted with urine then a higher temperature is detected there, which leads to a respective signal.

For the diaper and the method according to the invention, a diaper fastener with an active or passive sensor, especially an RFID element, can be used and can be particularly advantageous. The diaper fastener can additionally or alternatively comprise a temperature measuring device.

RFID tags can be made out of three pieces: a microchip (an integrated circuit which stores and processes information and modulates and demodulates radio-frequency (RF) signals), an antenna for receiving and transmitting the signal and a substrate. The tag information can be stored in a non-volatile memory. The RFID tag includes either fixed or programmable logic for processing the transmission and sensor data, respectively (see also Wikipedia in March 2020).

RFID tags can be either passive, active or battery-assisted passive. An active tag has an on-board battery and periodically transmits its ID signal. A battery-assisted passive has a small battery on board and is activated when in the presence of an RFID reader. A passive tag is cheaper and smaller because it has no battery; instead, the tag uses the radio energy transmitted by the reader.

Tags may either be read-only, having a factory-assigned serial number that is used as a key into a database, or may be read/write, where object-specific data can be written into the tag by the system user. Field programmable tags may be write-once, read-multiple; "blank" tags may be written with an electronic product code by the user.

The RFID tag receives the message and then responds with its identification and other information. This may be only a unique tag serial number, or may be product-related information such as a stock number, lot or batch number, production date, or other specific information. Since tags have individual serial numbers, the RFID system design can discriminate among several tags that might be within the range of the RFID reader and read them simultaneously.

RFID systems can be classified by the type of tag and reader. A Passive Reader Active Tag (PRAT) system has a passive reader which only receives radio signals from active tags (battery operated, transmit only). The reception range of a PRAT system reader can be adjusted from 1-2,000 feet (0-600 m), allowing flexibility in applications such as asset protection and supervision.

An Active Reader Passive Tag (ARPT) system has an active reader, which transmits interrogator signals and also receives authentication replies from passive tags.

An Active Reader Active Tag (ARAT) system uses active tags awoken with an interrogator signal from the active reader. A variation of this system could also use a Battery-Assisted Passive (BAP) tag which acts like a passive tag but has a small battery to power the tag's return reporting signal.

Fixed readers are set up to create a specific interrogation zone which can be tightly controlled. This allows a highly defined reading area for when tags go in and out of the interrogation zone. Mobile readers may be handheld or mounted on carts or vehicles.

The present invention will now be described with reference to the accompanying drawings, which illustrate embodiments of the invention. These embodiments should only exemplify, but not limit, the present invention.

### Figure Description

- Fig. 1: schematically exemplifies a system hardware architecture in accordance with the present invention;
- Fig. 2: schematically exemplifies a diaper according to an embodiment of the present invention;
- Fig. 3: schematically exemplifies a setup of a system according to an embodiment of the present invention; and
- Fig. 4: multiple rooms with patients and the monitoring setup according to the present invention.

### Description of preferred embodiments as exemplified in the figures

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

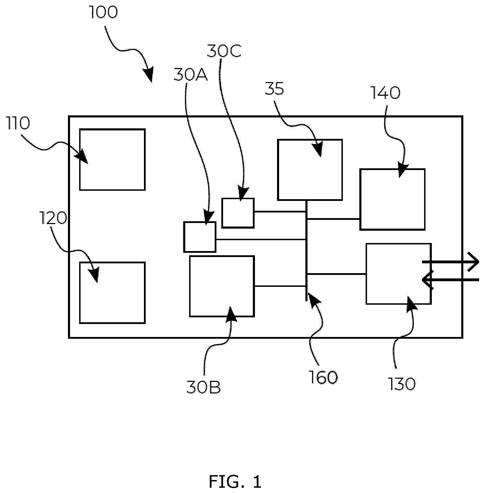

Fig. 1 provides a schematic of a computing device 100. The computing device 100 may comprise a computing unit 35, a first data storage unit 30A, a second data storage unit 30B and a third data storage unit 30C.

The computing device 100 can be a single computing device or an assembly of computing devices. The computing device 100 can be locally arranged or remotely, such as a cloud solution.

On the different data storage units 30 the different data can be stored, such as the genetic data on the first data storage 30A, the time stamped data and/or event code data and/or phenotypic data on the second data storage 30B and privacy sensitive data, such as the connection of the before-mentioned data to an individual, on the thirds data storage 30C.

Additional data storage can be also provided and/or the ones mentioned before can be combined at least in part. Another data storage (not shown) can comprise data specifying the composition or pharmaceutically active composition and/or medication data, such as pharmaceutical activities, side effects, interactions between the different components etc. This data can also be provided on one or more of the before-mentioned data storages.

The computing unit 35 can access the first data storage unit 30A, the second data storage unit 30B and the third data storage unit 30C through the internal communication channel 160, which can comprise a bus connection 160.

The computing unit 30 may be single processor or a plurality of processors, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The first data storage unit 30A may be singular or plural, and may be, but not limited to, a volatile or non-volatile memory, such as a random access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

The second data storage unit 30B may be singular or plural, and may be, but not limited to, a volatile or non-volatile memory, such as a random access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM). The third data storage unit 30C may be singular or plural, and may be, but not limited to, a volatile or non-volatile memory, such as a random access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

It should be understood that generally, the first data storage unit 30A (also referred to as encryption key storage unit 30A), the second data storage unit 30B (also referred to as data share storage unit 30B), and the third data storage unit 30C (also referred to as decryption key storage unit 30C) can also be part of the same memory. That is, only one general data storage unit 30 per device may be provided, which may be configured to store the respective encryption key (such that the section of the data storage unit 30 storing the encryption key may be the encryption key storage unit 30A), the respective data element share (such that the section of the data storage unit 30 storing the data element share may be the data share storage unit 30B), and the respective decryption key (such that the section of the data storage unit 30 storing the decryption key may be the decryption key storage unit 30A).

In some embodiments, the third data storage unit 30C can be a secure memory device 30C, such as, a self-encrypted memory, hardware-based full disk encryption memory and the like which can automatically encrypt all of the stored data. The data can be decrypted from the memory component only upon successful authentication of the party requiring to access the third data storage unit 30C, wherein the party can be a user, computing device, processing unit and the like. In some embodiments, the third data storage unit 30C can only be connected to the computing unit 35 and the computing unit 35 can be configured to never output the data received from the third data storage unit 30C. This can ensure a secure storing and handling of the encryption key (i.e. private key) stored in the third data storage unit 30C.

In some embodiments, the second data storage unit 30B may not be provided but instead the computing device 100 can be configured to receive a corresponding encrypted share from the database 60. In some embodiments, the computing device 100 may comprise the second data storage unit 30B and can be configured to receive a corresponding encrypted share from the database 60.

The computing device 100 may comprise a further memory component 140 which may be singular or plural, and may be, but not limited to, a volatile or non-volatile memory, such as a random access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM). The memory component 140 may also be connected with the other components of the computing device 100 (such as the computing component 35) through the internal communication channel 160.

Further the computing device 100 may comprise an external communication component 130. The external communication component 130 can be configured to facilitate sending and/or receiving data to/from an external device (e.g. backup device 10, recovery device 20, database 60). The external communication component 130 may comprise an antenna (e.g. WIFI antenna, NFC antenna, 2G/3G/4G/5G antenna and the like), USB port/plug, LAN port/plug, contact pads offering electrical connectivity and the like. The external communication component 130 can send and/or receive data based on a communication protocol which can comprise instructions for sending and/or receiving data. Said instructions can be stored in the memory component 140 and can be executed by the computing unit 35 and/or external communication component 130. The external communication component 130 can be connected to the internal communication component 160. Thus, data received by the external communication component 130 can be provided to the memory component 140, computing unit 35, first data storage unit 30A and/or second data storage unit 30B and/or third data storage unit 30C. Similarly, data stored on the memory component 140, first data storage unit 30A and/or second data storage unit 30B and/or third data storage unit 30C and/or data generated by the commuting unit 35 can be provided to the external communication component 130 for being transmitted to an external device.

In addition, the computing device 100 may comprise an input user interface 110 which can allow the user of the computing device 100 to provide at least one input (e.g. instruction) to the computing device 100. For example, the input user interface 110 may comprise a button, keyboard, trackpad, mouse, touchscreen, joystick and the like.

Additionally, still, the computing device 100 may comprise an output user interface 120 which can allow the computing device 100 to provide indications to the user. For example, the output user interface 110 may be a LED, a display, a speaker and the like.

The output and the input user interface 100 may also be connected through the internal communication component 160 with the internal component of the device 100.

The processor may be singular or plural, and may be, but not limited to, a CPU, GPU, DSP, APU, or FPGA. The memory may be singular or plural, and may be, but not limited to, being volatile or non-volatile, such an SDRAM, DRAM, SRAM, Flash Memory, MRAM, F-RAM, or P-RAM.

The data processing device can comprise means of data processing, such as, processor units, hardware accelerators and/or microcontrollers. The data processing device 20 can comprise memory components, such as, main memory (e.g. RAM), cache memory (e.g. SRAM) and/or secondary memory (e.g. HDD, SDD). The data processing device can comprise busses configured to facilitate data exchange between components of the data processing device, such as, the communication between the memory components and the processing components. The data processing device can comprise network interface cards that can be configured to connect the data processing device to a network, such as, to the Internet. The data processing device can comprise user interfaces, such as:
▪ output user interface, such as:
   o screens or monitors configured to display visual data (e.g. displaying graphical user interfaces of the questionnaire to the user),
   o speakers configured to communicate audio data (e.g. playing audio data to the user),
▪ input user interface, such as:
   ∘ camera configured to capture visual data (e.g. capturing images and/or videos of the user),
   o microphone configured to capture audio data (e.g. recording audio from the user),
   ∘ keyboard configured to allow the insertion of text and/or other keyboard commands (e.g. allowing the user to enter text data and/or other keyboard commands by having the user type on the keyboard) and/or trackpad, mouse, touchscreen, joystick - configured to facilitate the navigation through different graphical user interfaces of the questionnaire.

The data processing device can be a processing unit configured to carry out instructions of a program. The data processing device can be a system-on-chip comprising processing units, memory components and busses. The data processing device can be a personal computer, a laptop, a pocket computer, a smartphone, a tablet computer. The data processing device can be a server, either local and/or remote. The data processing device can be a processing unit or a system-on-chip that can be interfaced with a personal computer, a laptop, a pocket computer, a smartphone, a tablet computer and/or user interface (such as the upper-mentioned user interfaces).

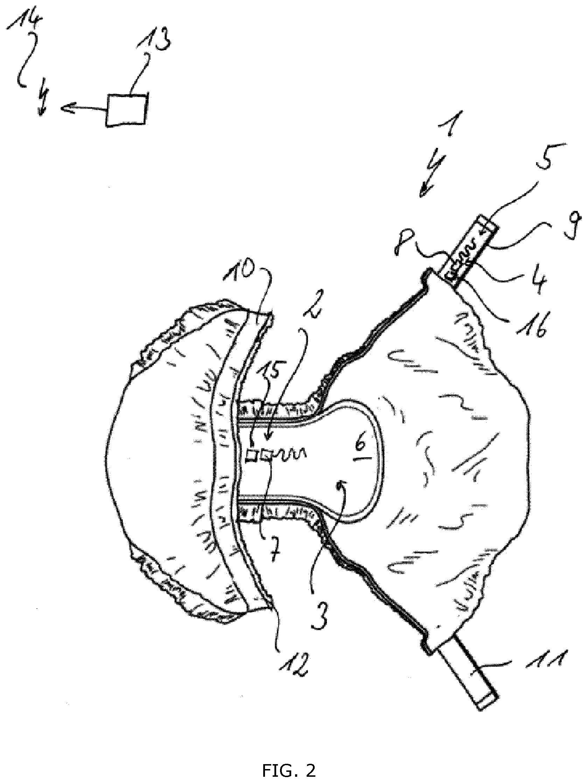

Fig. 2 shows a preferred example of a diaper and a system according to the invention. This figure shows a top view of an opened diaper. The diaper 1 shown in the figure shows a first transmitter 2 at a first location 3 of the diaper, which can get contaminated with excrements, particularly urine, when the diaper is in use.

A second transmitter 4 is mounted at a location 5, which, when using the diaper is not intended to get humid or wet. In the design example an insert 6, such as an absorption inlay 6, is provided in the bottom or crotch area of the diaper. On this part a transmitter 2, such as one comprising a passive sensor 2 and an RFID element 7, is provided. A second transmitter 4 comprises an RFID element 8, which can be printed or fastened in other ways on the diaper fastener 9.

The position of the two RFID elements 7, 8 facilitates that during humidification of the diaper 1 the first sensor 2 becomes moist and the second sensor 4 remains dry.

When the diaper or brief 1 is put on a person, the fastening 9 is adjusted to 10 and a further diaper fastener 11 is attached to a contact surface 12 glued thereon. Thus, the diaper fasteners are located outside the diaper so that the RFID element 8 is located at a location 5 which, when using the diaper 1 does not get wet.

If the RFID element 8 transmits data to a receiver 13 and thus indicates a dry situation and the RFID element 7 accordingly indicates a dry situation the receiver or any other device determines that the diaper is not damp. In this case no signal or alarm can be sent out.

However, if the RFID element 7 reports a moist condition or as a result of moisture does not report a signal, this can be concluded at receiver 13, that the diaper is wet. In this situation the receiver 13 sends a signal 14 that can be an alarm signal.

In addition to the RFID element 7, a temperature measuring device 15 is provided and a temperature measuring device 16 is provided in addition to the RFID element 8.

Additionally, the temperature measuring devices 15 and 16 are thus located at the first location 3 of the diaper 1, which can get wet with urine, and in a second place 5, which is intended to stay dry or uncontaminated with excrement.

Thus, the RFID elements can either be combined with a humidity and/or a temperature sensor and In case of a deviation of temperatures or humidity values or in the event of failure of one transmitter and in particular of the moist RFID element, the receiver 13 can send a signal 14 to trigger an alarm .

The first sensor 2 can be attached or integrated into the diaper 1 and the second sensor element 4 onto the outside of the diaper 1.

Since the diaper fastener 9 is usually designed as an adhesive strip onto which one RFID element can be printed, the second sensor 4 is on the diaper fastener. The temperature and/or humidity measuring devices 15, 16 can be integrated into the RFID element.

When using the diaper, a receiver continuously can send the signals of the first sensor element 2, which is located at the place 3 of the diaper 1, which gets wet by urine and a second sensor 4, which is mounted at the location 5m which does not get wet when using diaper 1.

In receiver 13, the difference between the signal strength of the first sensor 2 and the signal strength of the second sensor 4 and when reaching a threshold of difference, the signal 14 is triggered.

Correspondingly, the first temperature measuring device 15 can also be used to measure a temperature at the first location 3 of diaper 1 and, if a threshold is exceeded, the signal 14 is triggered.

It is particularly advantageous if the second temperature is measured at the second location 5 of the diaper 1 and if a threshold of the difference between the first and second temperature the signal 14 is reached, the signal 14 is triggered. This means that even if the first transmission element fails as a result of the wetness, a signal 14 is triggered, which indicates that it is wet.

According to a further development of the invention, the sensors can be attached to the diaper 1 either via a cable and/or wirelessly with each other or with other sensors.

Reference numbers and letters appearing between parentheses in the claims, identifying features described in the embodiments and illustrated in the accompanying drawings, are provided as an aid to the reader as an exemplification of the matter claimed. The inclusion of such reference numbers and letters is not to be interpreted as placing any limitations on the scope of the claims.

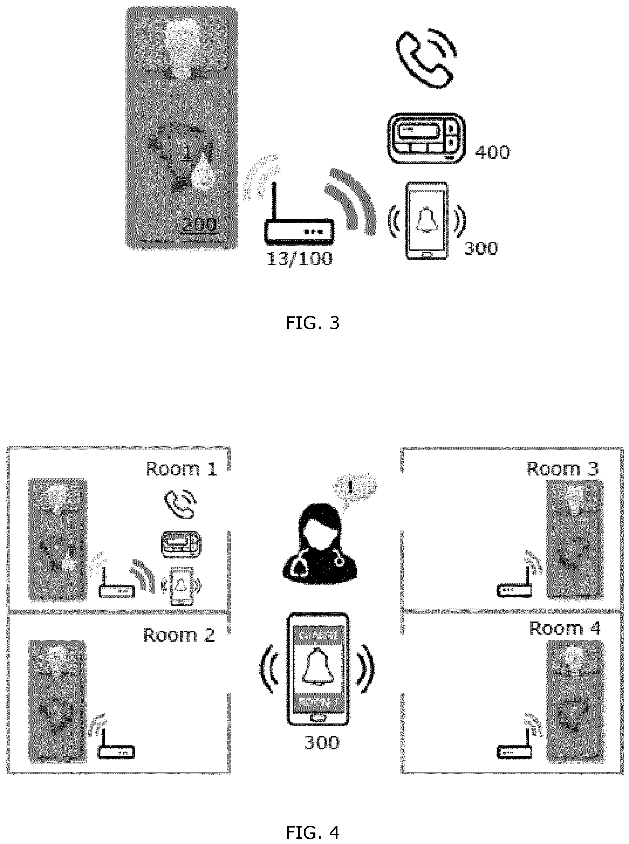

Fig. 3 shows a preferred setup in accordance with the present invention. A patient lying in a bed 200 is wearing a diaper 1. The wetness of the diaper is monitored by the receiver and/or processing unit 100. In case of wetness or excessive humidity the receiver and/or controlling unit 100 can activate an alarm signal to handheld devices 300, 400, such as a smartphone 300 or a landline phone 400 or other movable or fixed components.

A multi-room situation is exemplified in Fig. 4. An example of four rooms is provided. However, in practice many more rooms can be monitored as well. Anyhow in the example given, room 1 is sending an alarm in view of a wetted diaper or brief. A local alarm can be triggered by this or a central alarm or an alarm to a handheld device 300 with an indication where or in which room a diaper needs change.

The term "at least one of a first option and a second option" is intended to mean the first option or the second option or the first option and the second option.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. Diaper comprising a first transmitter at a first location that is configured to get contaminated with excrement, humidity and/or wetness, and that is configured to become inactive upon contamination with excrement, humidity and/or wetness, the diaper further comprising a second transmitter at a second location that is configured to stay uncontaminated with excrement, humidity and/or wetness, the diaper further comprising a processing component controlling at least one of the first transmitter and the second transmitter, wherein the processing component is configured to detect a difference between signals from the first transmitter and the second transmitter.

2. Diaper according to any one of the preceding claims wherein the first transmitter (2) is configured to submit a signal, in an uncontaminated status, in intervals.

3. Diaper according to the preceding claim wherein the interval is self-adjusting, according to the contamination of the transmitter (2).

4. Diaper according to any one of the preceding claims wherein the first transmitter (2) comprises a first RFID component (7).

5. Diaper according to any of the preceding claims wherein the first transmitter (2) comprises a humidity sensor (2) that is configured to be short-circuited upon contamination with excrement and/or a temperature sensor (15) and is configured to transmit data corresponding to a read-out of the humidity sensor (2) and/or the temperature sensor (15).

6. System for monitoring a diaper (1) comprising a diaper (1) according to any one of the preceding claims, the system comprising a receiver (13) configured to receive a signal from the diaper (1).

7. System according to the preceding claim wherein the receiver and the first transmitter are configured to read out and/or transmit an identification (ID) when, before and/or after the diaper is applied.

8. System according to any one of claims 6 or 7 wherein the receiver (13) comprises an RFID receiver, preferably an RFID excitation component that is configured to excite the transmitter(s) of the diaper comprising RFID(s).

9. System according to claim 8 wherein the RFID excitation component is configured to excite the first transmitter at any pre-set time interval.

10. System according to any of the preceding claims 6 to 9 further comprising a data processing device (100) that is configured to control the receiver (13) and/or to be controlled by the receiver (13) wherein the data processing device (100) is configured to control an alarm system and wherein the data processing device (100) is configured to activate the alarm system in case of a detection of excrement, humidity and/or wetness by the diaper (1).

11. System according to any of the preceding claims 6 to 10 further comprising an alarm deactivation device that is configured to deactivate, preferably automatically deactivate, an alarm upon change of the contaminated diaper (1).

12. System according to any one of the preceding claims 6 to 11 further comprising a monitoring system monitoring the number of diapers for a person per time unit.

13. System according to any one of the preceding claims 6 to 12 further comprising a monitoring system setting a monitoring alarm when the need for the change of diapers exceeds a threshold number of diapers for a person over time.

14. Method of monitoring a diaper comprising a first transmitter at a first location that is configured to get contaminated with excrement, humidity and/or wetness, and that is configured to become inactive upon contamination with excrement, humidity and/or wetness, the diaper further comprising a second transmitter at a second location that is configured to stay uncontaminated with excrement, humidity and/or wetness, the diaper further comprising a processing component controlling at least one of the first transmitter and the second transmitter, wherein the processing component is configured to detect a difference between signals from the first transmitter and the second transmitter.

15. Method according to the preceding claim with the steps of receiving the signal from the diaper by a receiver configured to receive any signals from the diaper, further comprising a data processing device that is configured to control the receiver and/or to be controlled by the receiver, wherein the data processing device is configured to activate an alarm system in case of a detection of excrement, humidity and/or wetness of the diaper.

## Patentansprüche

1. Windel, die einen ersten Sender an einer ersten Stelle umfasst, der dazu konfiguriert ist, mit Exkrementen, Feuchtigkeit und/oder Nässe verunreinigt zu werden, und der dazu konfiguriert ist, bei Verunreinigung mit Exkrementen, Feuchtigkeit und/oder Nässe inaktiv zu werden, wobei die Windel ferner einen zweiten Sender an einer zweiten Stelle umfasst, der dazu konfiguriert ist, nicht mit Exkrementen, Feuchtigkeit und/oder Nässe verunreinigt zu werden, wobei die Windel ferner eine Verarbeitungskomponente umfasst, die den ersten Sender und/oder den zweiten Sender steuert, wobei die Verarbeitungskomponente dazu konfiguriert ist, einen Unterschied zwischen den Signalen von dem ersten Sender und dem zweiten Sender zu erfassen.

2. Windel nach einem der vorhergehenden Ansprüche, wobei der erste Sender (2) dazu konfiguriert ist, in einem nicht verunreinigten Zustand in Intervallen ein Signal abzugeben.

3. Windel nach dem vorhergehenden Anspruch, wobei das Intervall entsprechend der Verunreinigung des Senders (2) selbsteinstellend ist.

4. Windel nach einem der vorhergehenden Ansprüche, wobei der erste Sender (2) eine erste RFID-Komponente (7) umfasst.

5. Windel nach einem der vorhergehenden Ansprüche, wobei der erste Sender (2) einen Feuchtigkeitssensor (2), der dazu konfiguriert ist, bei Verunreinigung mit Exkrementen kurzgeschlossen zu werden, und/oder einen Temperatursensor (15) umfasst und dazu konfiguriert ist, Daten entsprechend einem Auslesen des Feuchtigkeitssensors (2) und/oder des Temperatursensors (15) zu senden.

6. System zur Überwachung einer Windel (1), umfassend eine Windel (1) nach einem der vorhergehenden Ansprüche, wobei das System einen Empfänger (13) umfasst, der dazu konfiguriert ist, ein Signal von der Windel (1) zu empfangen.

7. System nach dem vorhergehenden Anspruch, wobei der Empfänger und der erste Sender dazu konfiguriert sind, eine Identifikation (ID) auszulesen und/oder zu senden, wenn, vor und/oder nach dem die Windel angelegt wird.

8. System nach einem der Ansprüche 6 oder 7, wobei der Empfänger (13) einen RFID-Empfänger, vorzugsweise eine RFID-Anregungskomponente, umfasst, die dazu konfiguriert ist, den/die Sender der Windel, welche RFID(s) umfasst, anzuregen.

9. System nach Anspruch 8, wobei die RFID-Anregungskomponente dazu konfiguriert ist, den ersten Sender in einem beliebigen voreingestellten Zeitintervall anzuregen.

10. System nach einem der vorhergehenden Ansprüche 6 bis 9, das ferner eine Datenverarbeitungsvorrichtung (100) umfasst, die dazu konfiguriert ist, den Empfänger (13) zu steuern und/oder von dem Empfänger (13) gesteuert zu werden, wobei die Datenverarbeitungsvorrichtung (100) dazu konfiguriert ist, ein Alarmsystem zu steuern, und wobei die Datenverarbeitungsvorrichtung (100) dazu konfiguriert ist, das Alarmsystem im Falle einer Erfassung von Exkrementen, Feuchtigkeit und/oder Nässe durch die Windel (1) zu aktivieren.

11. System nach einem der vorhergehenden Ansprüche 6 bis 10, das ferner eine Alarmdeaktivierungsvorrichtung umfasst, die dazu konfiguriert ist, einen Alarm beim Wechseln der verunreinigten Windel (1) zu deaktivieren, vorzugsweise automatisch zu deaktivieren.

12. System nach einem der vorhergehenden Ansprüche 6 bis 11, das ferner ein Überwachungssystem umfasst, das die Anzahl der Windeln für eine Person pro Zeiteinheit überwacht.

13. System nach einem der vorhergehenden Ansprüche 6 bis 12, das ferner ein Überwachungssystem umfasst, das einen Überwachungsalarm auslöst, wenn der Bedarf an Windelwechseln eine Schwellenzahl von Windeln für eine Person im Laufe der Zeit überschreitet.

14. Verfahren zum Überwachen einer Windel, die einen ersten Sender an einer ersten Stelle umfasst, der dazu konfiguriert ist, mit Exkrementen, Feuchtigkeit und/oder Nässe verunreinigt zu werden, und der dazu konfiguriert ist, bei Verunreinigung mit Exkrementen, Feuchtigkeit und/oder Nässe inaktiv zu werden, wobei die Windel ferner einen zweiten Sender an einer zweiten Stelle umfasst, der dazu konfiguriert ist, nicht mit Exkrementen, Feuchtigkeit und/oder Nässe verunreinigt zu werden, wobei die Windel ferner eine Verarbeitungskomponente umfasst, die den ersten Sender und/oder den zweiten Sender steuert, wobei die Verarbeitungskomponente dazu konfiguriert ist, einen Unterschied zwischen den Signalen von dem ersten Sender und dem zweiten Sender zu erfassen.

15. Verfahren nach dem vorhergehenden Anspruch mit den Schritten zum Empfangen des Signals von der Windel durch einen Empfänger, der dazu konfiguriert ist, jegliche Signale von der Windel zu empfangen, ferner umfassend eine Datenverarbeitungsvorrichtung, die dazu konfiguriert ist, den Empfänger zu steuern und/oder von dem Empfänger gesteuert zu werden, wobei die Datenverarbeitungsvorrichtung dazu konfiguriert ist, ein Alarmsystem im Falle einer Erfassung von Exkrementen, Feuchtigkeit und/oder Nässe der Windel zu aktivieren.

## Revendications

1. Couche comprenant un premier émetteur à un premier emplacement qui est configuré pour être contaminé par des excréments, de l'humidité et/ou de la mouillure, et qui est configuré pour devenir inactif en cas de contamination par des excréments, de l'humidité et/ou de la mouillure, la couche comprenant en outre un second émetteur à un second emplacement qui est configuré pour ne pas être contaminé par des excréments, de l'humidité et/ou de la mouillure, la couche comprenant en outre un composant de traitement commandant au moins un du premier émetteur et du second émetteur, le composant de traitement étant configuré pour détecter une différence entre des signaux provenant du premier émetteur et du second émetteur.

2. Couche selon l'une quelconque des revendications précédentes, dans laquelle le premier émetteur (2) est configuré pour soumettre un signal, dans un état non contaminé, par intervalles.

3. Couche selon la revendication précédente, dans laquelle l'intervalle est auto-ajusté, en fonction de la contamination de l'émetteur (2).

4. Couche selon l'une quelconque des revendications précédentes, dans laquelle le premier émetteur (2) comprend un premier composant RFID (7).

5. Couche selon l'une quelconque des revendications précédentes, dans laquelle le premier émetteur (2) comprend un capteur d'humidité (2) configuré pour être court-circuité en cas de contamination par des excréments et/ou un capteur de température (15) et est configuré pour transmettre des données correspondant à une lecture du capteur d'humidité (2) et/ou du capteur de température (15).

6. Système de surveillance d'une couche (1) comprenant une couche (1) selon l'une quelconque des revendications précédentes, le système comprenant un récepteur (13) configuré pour recevoir un signal provenant de la couche (1).

7. Système selon la revendication précédente, dans lequel le récepteur et le premier émetteur sont configurés pour lire et/ou transmettre une identification (ID) pendant, avant et/ou après une mise en place de la couche.

8. Système selon l'une quelconque des revendications 6 ou 7, dans lequel le récepteur (13) comprend un récepteur RFID, de préférence un composant d'excitation RFID qui est configuré pour exciter le ou les émetteurs de la couche comprenant un ou plusieurs RFID.

9. Système selon la revendication 8, dans lequel le composant d'excitation RFID est configuré pour exciter le premier émetteur à n'importe quel intervalle de temps prédéfini.

10. Système selon l'une quelconque des revendications précédentes 6 à 9, comprenant en outre un dispositif de traitement de données (100) qui est configuré pour commander le récepteur (13) et/ou pour être commandé par le récepteur (13), le dispositif de traitement de données (100) étant configuré pour commander un système d'alarme et le dispositif de traitement de données (100) étant configuré pour activer le système d'alarme dans le cas d'une détection d'excréments, d'humidité et/ou de mouillure par la couche (1).

11. Système selon l'une quelconque des revendications précédentes 6 à 10, comprenant en outre un dispositif de désactivation d'alarme qui est configuré pour désactiver, de préférence automatiquement, une alarme lors d'un changement de la couche contaminée (1).

12. Système selon l'une quelconque des revendications précédentes 6 à 11, comprenant en outre un système de surveillance surveillant le nombre de couches pour une personne par unité de temps.

13. Système selon l'une quelconque des revendications précédentes 6 à 12, comprenant en outre un système de surveillance déclenchant une alarme de surveillance lorsque le besoin de changement de couches dépasse un nombre seuil de couches pour une personne au cours du temps.

14. Procédé de surveillance d'une couche comprenant un premier émetteur à un premier emplacement qui est configuré pour être contaminé par des excréments, de l'humidité et/ou de la mouillure, et qui est configuré pour devenir inactif en cas de contamination par des excréments, de l'humidité et/ou de la mouillure, la couche comprenant en outre un second émetteur à un second emplacement qui est configuré pour ne pas être contaminé par des excréments, de l'humidité et/ou de la mouillure, la couche comprenant en outre un composant de traitement commandant au moins un du premier émetteur et du second émetteur, le composant de traitement étant configuré pour détecter une différence entre des signaux provenant du premier émetteur et du second émetteur.

15. Procédé selon la revendication précédente, avec les étapes de réception du signal provenant de la couche par un récepteur configuré pour recevoir tout signal provenant de la couche, comprenant en outre un dispositif de traitement de données qui est configuré pour commander le récepteur et/ou pour être commandé par le récepteur, le dispositif de traitement de données étant configuré pour activer un système d'alarme dans le cas d'une détection d'excréments, d'humidité et/ou de mouillure de la couche.
